# EUROPÄISCHE PATENTSCHRIFT

(11) **EP 0 475 980 B1**
(45) Veröffentlichungstag und Bekanntmachung des Hinweises auf die Patenterteilung: **10.11.1993**
(21) Anmeldenummer: 90908507.8
(22) Anmeldetag: 07.06.1990
(51) Int. Cl.: A61B 17/36, A61B 17/22, B23K 26/04

(54) **VERFAHREN UND VORRICHTUNG ZUR MATERIALBEARBEITUNG MIT HILFE EINES LASERS**
PROCESS AND DEVICE FOR TREATING A MATERIAL WITH A LASER
PROCEDE ET DISPOSITIF DE TRAITEMENT D'UNE SUBSTANCE A L'AIDE D'UN LASER

(30) Priorität: 07.06.1989 DE 3918618
(43) Veröffentlichungstag der Anmeldung: 25.03.1992
(73) Patentinhaber: Telemit Electronic GmbH, 80906 München (DE)
(72) Erfinder: ENGELHARDT, Ralf, D-3162 Uetze (DE); BEAUCAMP, Danielle, D-8000 München 40 (DE); SCHEU, Manfred, D-2400 Lübeck (DE)
(74) Vertreter: Feldkamp, Rainer, Dipl.-Ing.
(86) Internationale Anmeldenummer: EP9000895
(87) Internationale Veröffentlichungsnummer: WO9014797

(56) Entgegenhaltungen:
- EP-A- 0 312 650
- WO-A-86/06269
- WO-A-88/08279
- US-A- 4 785 806

## Beschreibung

Die Erfindung bezieht sich auf ein Verfahren sowie eine Vorrichtung zur Materialbearbeitung mit Hilfe eines Lasers der im Oberbegriff des Anspruchs 1 bzw. 3 genannten Art.

Es ist ein Verfahren bekannt (Optical Studies of Pulsed-Laser Fragmentation of Biliary Calculi, Applied Physics B, Springer Verlag 1987 Seiten 73-78), bei dem das Ausgangssignal eines Lasers über eine einen Lichtwellenleiter einschließende Laseroptik auf Harn- oder Gallensteine gerichtet wird, um diese zu zertrümmern. In der Laseroptik ist ein halbdurchlässiger Spiegel angeordnet, der einen Teil des von dem Stein reemittierten, zurückreflektierten oder zurückgestreuten Lichtes, das über den Lichtwellenleiter zurückgeleitet wird, auf einen Detektor (1) leitet, dem eine Auswerteschaltung in Form eines Spektralanalysators nachgeschaltet ist.

Es ist weiterhin bekannt (EP-A2-01 95 375), bei der Entfernung von Ablagerungen auf Geweben den Bearbeitungsbereich mit einem Pilotlaser geringer Leistung zu bestrahlen und die reemittierte Energie bei beispielsweise drei Wellenlängen auszuwerten, um festzustellen, ob das von dem Pilotlaser getroffene Material durch eine Ablagerung oder durch Gewebe gebildet ist, das nicht von einem Bearbeitungslaserimpuls getroffen werden soll. Hierbei ist jedoch ein getrennter Pilotlaser erforderlich, der vor dem Auslösen des Bearbeitungslaserimpulses zur Feststellung des vor der Laseroptik liegenden Materials verwendet wird.

Schließlich ist ein Verfahren bzw. eine Vorrichtung der eingangs genannten Art bekannt (EP-A1-0 312 650), bei dem bzw. bei der lediglich ein einziger Laser verwendet wird, dessen Laserimpuls vor dem Erreichen der vollen Leistung unterbrochen oder zumindestens in seiner Leistung verringert werden kann, wenn während des Anstiegs des Laserimpulses durch eine Auswertung des von dem Material zurückkehrenden Lichtes festgestellt wird, daß dieser Laserimpuls auf Gewebe und nicht auf zu entfernendes Material auftrifft.

Der Erfindung liegt die Aufgabe zugrunde, ein Verfahren sowie eine Vorrichtung der eingangs genannten Art zu schaffen, bei dem bzw. bei der bei geringem Aufwand eine Identifizierung des zu bearbeitenden Materials ermöglicht wird.

Diese Aufgabe wird durch die im kennzeichnenden Teil des Patentanspruchs 1 bzw. 3 angegebenen Merkmale gelöst. Vorteilhafte Ausgestaltungen und Weiterbildungen der Erfindung ergeben sich aus den jeweiligen Unteransprüchen.

Bei dem erfindungsgemäßen Verfahren bzw. der Vorrichtung wird zur Steuerung der Energie des Lasers in Abhängigkeit von dem Auftreffen des Laserstrahls auf zu bearbeitendes Material eine Identifizierung der Zusammensetzung des bearbeiteten Materials zu einem Zeitpunkt während des Zeitraumes durchgeführt, der zwischen der Auslösung des Laserimpulses und dem durch den Laserimpuls hervorgerufenen dielektrischen Durchbruch auf dem zu bearbeitenden Material liegt. Das zu diesem Zeitpunkt reemittierte Licht wird hinsichtlich der Intensität in zumindestens zwei vorgegebenen Spektralbereichen durch Quotientenbildung ausgewertet, wobei diese Auswertung mit Hilfe einer Detektoranordnung mit auf die zumindestens zwei Spektralbereiche abgestimmten Detektoren erfolgt.

Das Ergebnis dieser Identifizierung kann zur Steuerung der Energie der Bearbeitungsimpulse derart herangezogen werden, daß sich eine optimale Materialabtragung ergibt.

Vor der Detektoranordnung ist vorzugsweise eine Filteranordnung angeordnet, die genau die Laserlinie ausblendet, sodaß der Nachweis von Materialspektren ermöglicht wird, die im längerwelligen Bereich neben der Laserlinie liegen.

Erfindungsgemäß wurde festgestellt, daß sich die Spektren des Lichtes, das im Verlauf der Anstiegsflanke des Laserimpulses beispielsweise von unterschiedlichen Steintypen oder von Materialablagerungen in Blutgefäßen im menschlichen Körper reemittierten wird, hinsichtlich der Form und der Lage der Maxima unterscheiden, so daß die Slgnalstärken am Ausgang der zumindestens zwei auf die vorgegebenen Spektralbereiche abgestimmten Detektoren der Detektoranordnung die Identifizierung des bearbeiteten Materials ermöglicht, wobei die Ausgangssignale zu einem oder mehreren vorherbestimmten Zeitpunkten nach der Auslösung des Laserimpulses ausgewertet werden.

Die Ausgangssignale der mindestens zwei auf unterschiedliche vorgegebene Spektralbereiche abgestimmten Detektoren in der Detektoranordnung werden hierbei durch eine Quotientenbildung ausgewertet, um die Zusammensetzung beispielsweise von Steinen oder Ablagerungen zu bestimmen.

Hierbei kann gemäß einer Ausführungsform der Erfindung das reemittierte Licht über auf unterschiedliche Spektralbereiche abgestimmte Bandpaßfilter auf getrennte Lichtdetektoren, beispielsweise Fotodioden geleitet werden, deren Ausgangssignale zu dem vorgegebenen Zeitpunkt ins Verhältnis zueinander gesetzt werden, wobei das Ergebnis dieser Quotientenbildung zu dem vorgegebenen Zeitpunkte zur Bestimmung der Art und Zusammensetzung des von dem Laserlicht getroffenen Materials verwendet wird.

Vorzugsweise werden die Ausgangssignale der einzelnen Detektoren bzw. des Detektors der Detektoranordnung bei den verschiedenen Spektralbereichen mit Hilfe des Ausgangssignals eines das Gesamtspektrum des reemittierten Lichtes erfassenden weiteren Detektors normiert, worauf eine Auswertung erfolgt, die eine eindeutige Aussage über das von dem Laserimpuls getroffene Materials ermöglicht.

Das erfindungsgemäße Verfahren und die Vorrichtung eignet sich damit sowohl zur Lithrotripsie als auch zur Angioplastie, und es sind weiterhin auch technische Anwendungen, beispielsweise bei der Herstellung von Halbleitern und gedruckten Schaltungen denkbar.

Durch die gleichzeitige Verwendung des Laserlmpulses sowohl zur Bearbeitung als auch zur Identifizierung des Materials ist kein getrennter Pilotlaser zur Bestimmung des Materials erforderlich.

Die Erfindung wird im folgenden anhand von in der Zeichnung dargelegten Ausführungsbeispielen noch näher erläutert.

In der Zeichnung zeigen:
- Fig. 1: eine Ausführungsform einer Vorrichtung zur Durchführung des Verfahrens,
- Fig. 2: Diagramme, die Beispiele für einen zeitlichen Verlauf des auf die Detektoranordnung einfallenden Lichtes zeigen,
- Fig. 3: eine Darstellung der spektralen Energieverteilung des von drei verschiedenen Steintypen reemittierten Lichtes in dem in Fig. 2 mit A markierten Zeitraum,
- Fig. 4: eine weitere Ausführungsform einer Vorrichtung zur Durchführung des Verfahrens, insbesonder zur Angioplastie,
- Fig. 5: eine Oszillograph-Darstellung der mit der Vorrichtung nach Fig. 4 gewonnenen Meßergebnisse, wobei das Laserlicht auf Kalkablagerungen auftrifft und die Laserenergie oberhalb des Schwellwertes für eine Plasmabildung liegt,
- Fig. 6a und 6b: eine der Fig. 5 entsprechende Darstellung für den Fall des Auftreffens von Laserlicht mit einer Energie unterhalb des Plasmaschwellwertes beim Auftreffen auf Kalkablagerungen bzw. auf normales Gewebe,
- Fig. 7: eine Darstellung des Verlaufes des Laserimpulses beim Auftreffen auf Kalkablagerungen bzw. auf normales Gewebe unter Steuerung durch die Ausführungsform der Vorrichtung nach Fig. 1 oder Fig. 4.

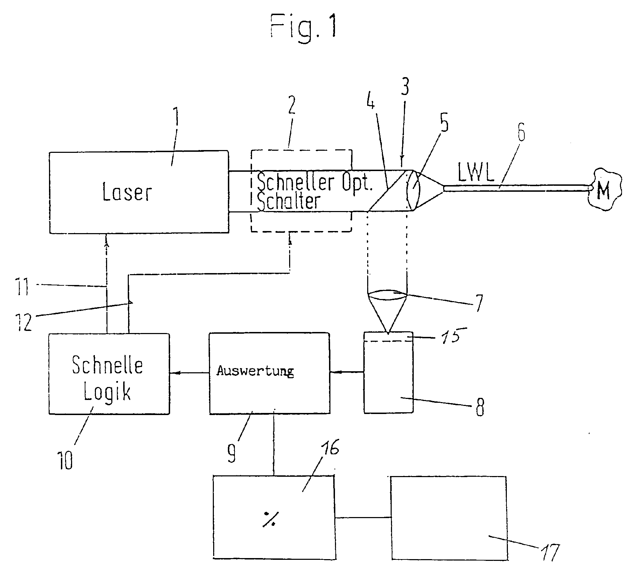

Die in Fig. 1 dargestellte Ausführungsform einer Vorrichtung zur Durchführung des Verfahrens weist einen Laser (1) auf, dessen Ausgangsimpulse über einen eine hohe Schaltgeschwindigkeit aufweisenden optischen Schalter (2), z.B. eine Pockel-Zelle, einer Laseroptik (3) zugeführt werden, deren Ausgangsstrahlung über einen Lichtwellenleiter (6) auf zu bearbeitendes Material (M) geric htet wird. Bei der dargestellten Ausführungsform weist die Laseroptik (3) beispielsweise einen Strahlteiler (4) sowie eine erste Linse (5) zur Fokussierung des Laserlichtimpulses auf den Lichtwellenleiter (6) auf. Von dem zu bearbeitenden Material (M) reemittiertes Licht gelangt über den Lichtwellenleiter (6), die Linse (5) und den Strahlteiler (4) auf eine Linse (7), die dieses Licht über ein Filter (15) auf eine Detektoranordnung (8) fokussiert. Das Ausgangssignal wird einer noch näher zu erläuternden Auswerteschaltung (9) zugeführt, die über Logikschaltungen (10) den optischen Schalter (2) über eine Ausgangsleitung (12) steuert. Wie dies durch eine weitere Ausgangsleitung (11) der Logikschaltung (10) angedeutet ist, kann diese auch den Laser (1) selbst steuern, wobei ggf. der optische Schalter (2) entfallen kann.

Bei Auftreffen eines Laserimpulses hoher Energie auf das zu bearbeitende Material (M) wird in unmittelbarer Nähe dieses zu bearbeitenden Materials ein dielektrischer Durchbruch erzeugt, der eine Schockwelle zur Bearbeitung des Materials auslöst.

Dieses Material kann beispielsweise ein menschlicher Stein, beispielsweise ein Harn- oder Gallenstein, der in umgebendes Gewebe eingebettet ist, oder eine Materialablagerung auf Gewebe sein. Die durch die Laserstrahlung hervorgerufene Plasmablase bewirkt durch die hohe Temperatur des Plasmas und die hierbei entstehende Druckwelle eine Materialabsprengung, -abtragung oder -aufspaltung am Zielort. Hierbei muß jedoch in vielen Fällen, insbesondere bei dem betrachteten Fall der Zertrümmerung von menschlichen Steinen oder bei der Angioplastie darauf geachtet werden, daß der am Ende des Lichtwellenleiters (6) austretende Laserimpuls lediglich auf das zu bearbeitende Material (M), nicht jedoch auf das umgebende Gewebe auftrifft, damit dieses nicht zerstört wird.

Die Detektoranordnung (8) ermöglicht in Verbindung mit der Auswerteschaltung (9) eine sehr schnelle Ermittlung, ob zu bearbeitendes Material am Ende des Lichtwellenleiters (6) angeordnet ist, oder ob dieses Ende des Lichtwellenleiters auf Material gerichtet ist, das nicht mit dem Laserimpuls, zumindestens nicht mit einem Laserimpuls voller Leistung beaufschlagt werden darf.

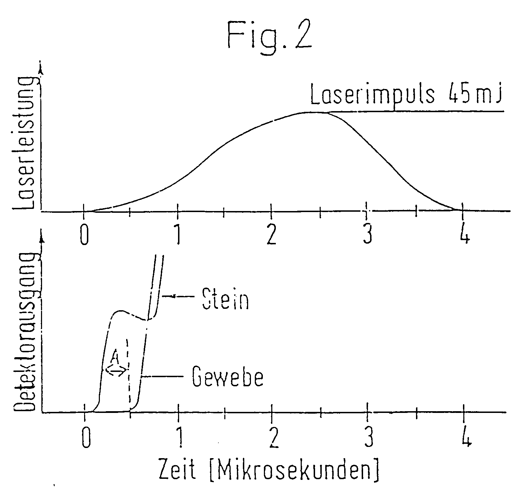

Zu diesem Zweck wertet die Auswerteschaltung den zeitlichen Funktionsverlauf des auf die Detektoranordnung einfallenden Lichtes bezogen auf den zeitlichen Verlauf des Laserimpulses aus, wobei die Art dieser Auswertung anhand der Fig. 2 näher erläutert wird.

Im oberen Teil dieser Fig. 2 ist der Verlauf des Laserimpulses dargestellt. Im unteren Teil der Fig. 2 ist das von dem Material (M) reemittierte Licht in Form des Ausgangssignals der Detektoranordnung dargestellt. Dieses reemittierte Licht ergibt sich aus der durch den Laserstrahl in dem Material ausgelöste Fluoreszenz.

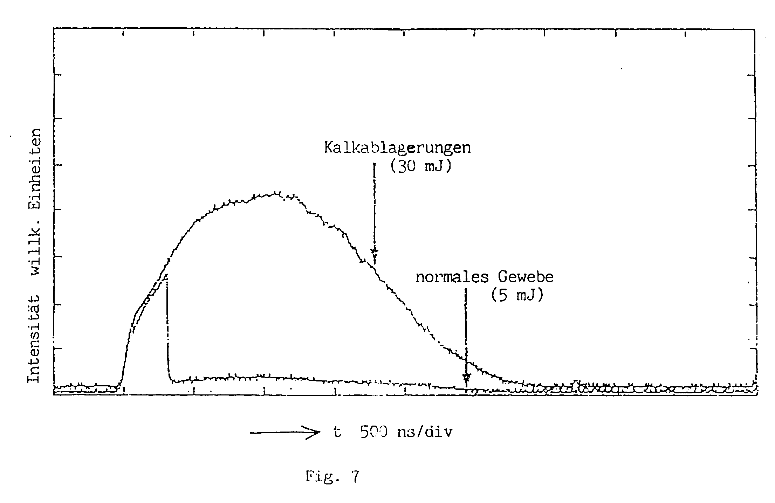

Wie aus dem unteren Teil der Fig. 2 zu erkennen ist, steigt das Ausgangssignal der Detektoranordnung bei Auftreffen des Laserlichtes auf einen Stein bereits nach etwa 100 bis 150 ns an, während bei Auftreffen auf Gewebe dieser Anstieg erst etwa 500 ns nach Auslösen des Laserimpulses erfolgt. Zu diesem Zeitpunkt hat der Laserimpuls seine volle Leistung noch nicht erreicht, sodaß es über die Leitung (11) und/oder die Leitung (12) mit Hilfe des schnellen optischen Schalters (2) nach Fig. 1 möglich ist, den am Ende des Lichtwellenleiters (6) austretenden Laserimpuls vor Erreichen der maximalen Energie zu beenden, wie dies anhand der Fig. 7 noch näher erläutert wird.

Im unteren Teil der Fig. 2 ist weiterhin mit A ein Zeitraum bezeichnet, der in vorteilhafter Weise zur Identifizierung des zu bearbeitenden Materials verwendet werden kann. Beispielsweise ist es in diesem Fall möglich, die chemische Zusammensetzung, die Struktur und die physikalischen Eigenschaften von Harn-, Gallen- oder anderen Steinen oder von Kalkablagerungen auf Blutgefäßen im menschlichen Körper ohne die Verwendung eines zusätzlichen Hilfs- oder Pilotlasers zu identifizieren.

Dieser Zeitraum erstreckt sich in Abhängigkeit von dem Lasertyp und dgl. über einen Bereich von etwa 500 ns 1,2 µs und liegt zwischen dem Zeitpunkt des Auslösens des Laserimpulses und dem dielektrischen Durchbruch an dem zu bearbeitenden Material.

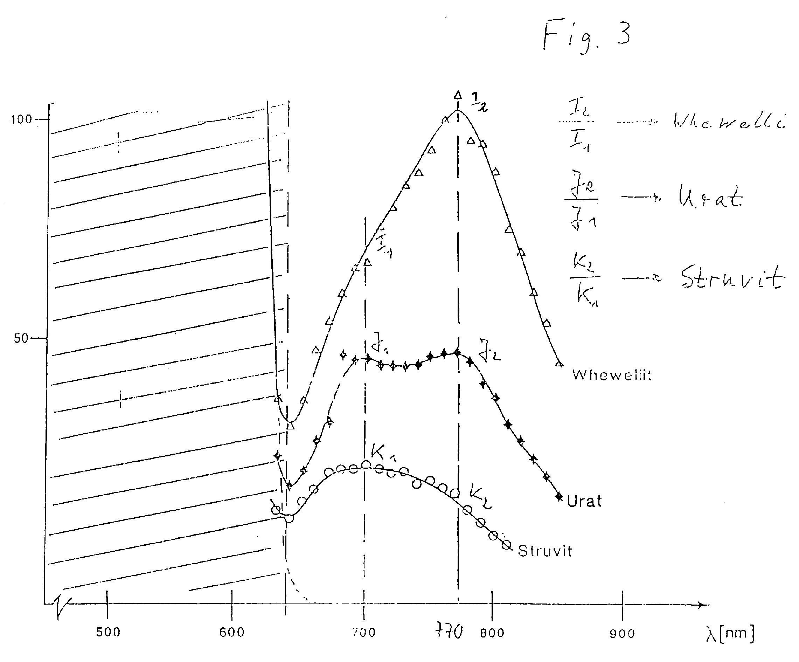

Zu diesem Zweck wird das vom Stein reemittierte Licht spektral zerlegt registriert, und als Beispiel sind in Fig. 3 drei von Harnsteinen unterschiedlicher Zusammensetzung gewonnene Spektren gezeigt. Es handelt sich hierbei je um einen Struvit-, einen Whewellit- und einen Uratstein. Die Spektren unterscheiden sich in der Form und in der Lage der Maxima und können leicht durch Auswertung der Intensität des reemittierten Lichtes bei mindestens einem vorgegebenen Spektalbereich innerhalb des Gesamt-Spektralbereiches dieses reemittierten Lichtes oder durch Quotientenbildung der normierten Signalstärken im Beispiel der Figur 3 bei 700 nm und 770 nm unterschieden werden. In diesem Beispiel erfolgt die Anregung bei einer Wellenlänge von etwa 600 nm.

Die Vorrichtung nach Fig. 1 weist eine der Detektoranordnung und der Auswerteschaltung nachgeschaltet Rechenschaltung (16) auf, die im Zeitraum vor dem Zünden des Plasmas den Quotienten der Ausgangssignale von Detektoren der Detektoranordnung (8) bei zwei unterschiedlichen Spektralbereichen bildet und diesen an einer Darstellungsvorrichtung (17) anzeigt.

Das Ausgangssignal der Auswerteschaltung (9) wird zur Steuerung der Energie und/oder der Dauer der Bearbeitungsimpulse in Abhängigkeit von dem identifizierten Material verwendet.

Das von dem Material reemittierte Licht wird über den Strahlteiler (4) über das Filter (15) auf die Detektoranordnung geleitet, wobei das Filter genau die Laserlinie ausblendet, während die Materialspektren, die im längerwelligen Bereich neben der Laserlinie liegen, ausgewertet werden können.

Die Detektoranordnung umfaßt zumindestens zwei Detektoren, die auf Licht mit unterschiedlichen Spektralbereichen ansprechen.

Weiterhin kann die Detektoranordnung (8) einen zusätzlichen Detektor einschließen, der das Gesamtspektrum des reemittierten Lichtes erfaßt und ein Ausgangssignal liefert, das zur Normierung des Ausgangssignals oder der Ausgangssignale bei den einzelnen Spektralbereichen in der Auswerteschaltung (9) verwendet wird.

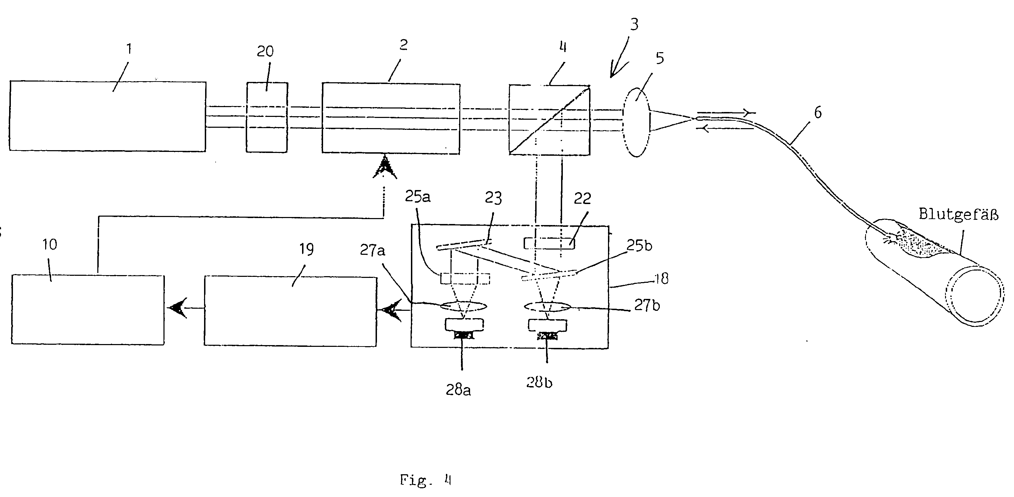

In Fig. 4 ist eine weitere Ausführungsform der Vorrichtung zur Durchführung des Verfahrens gezeigt, die sich insbesondere für die Angioplastie eignet, obwohl die nachfolgend beschriebenen Einzelheiten der Auswerteschaltung auch für die Bearbeitung anderer Materialien geeignet sind.

Die Ausführungsform nach Fig. 4 weist ebenso wie die in Fig. 1 dargestellte Ausführungsform einen Laser (1) auf, der beispielsweise ein blitzlichtgepumpter Farbstofflaser sein kann. Das Ausgangssignal des Lasers (1) wird einem optischen Schalter (2) vorzugsweise über ein Farbstofffluorenszenz-Sperrfilter (20) zur Unterdrückung störender Farbstofffluoreszenz im interessierenden Wellenbereich zugeführt, was es ermöglicht, einen einzigen Lichtleiter (6) sowohl zur Übertragung des Laserimpulses an das zu bearbeitende Material als auch zur Rückleitung des reemittierten Lichtes zu verwenden.

Auf den schnellen optischen Schalter (2), beispielsweise in Form einer Pockel-Zelle, folgt wiederum eine Laseroptik (3) mit einen Strahlteiler, der vorzugsweise ein polarisierender Strahlteiler (4) ist, und mit einer Linse (5), die das Laserlicht auf das Eingangsende des Lichtwellenleiters (6) fokussiert. Das freie Ende des Lichtwellenleiters (6) ist als Beispiel in einem Blutgefäß angeordnet, um dort Kalk- oder Fettablagerungen zu beseitigen.

Das in das freie Ende des Lichtwellenleiters reemittierte, durch die Fluoreszenz des von dem Laserstrahl getroffenen Materials erzeugte Licht gelangt über den polarisierenden Strahlteiler (4) auf ein die Laserwellenlänge unterdrückendes Filter (22) und von dort auf ein erstes Bandpaßfilter, das lediglich reemittiertes Licht mit einem ersten Spektralbereich durchläßt und über eine Linse (27b) auf einen ersten Fotodetektor (28b) fokussiert. Das Bandpaßfilter (25b) ist teildurchlässig, so daß ein Teil des auf dieses Bandpaßfilter auftreffendes Lichtes auf einen Spiegel (23) reflektiert wird, der das Licht auf ein zweites Bandpaßfilter (25a) mit einem anderen Spektral-Durchlaßbereich als das Filter (25b) lenkt. Das das Bandpaßfilter (25a) passierende Licht wird wiederum über eine Linse (27a) auf einen zweiten Fotodetektor (28a) fokussiert.

Die Ausgangssignale der beiden Fotodetektoren werden einer Auswerte- und Anzeigeschaltung (19) zugeführt, deren Auswerteteil beipielsweise schnelle elektronische Vergleicher oder andere Rechenglieder enthalten kann. Der Anzeigeteil kann beispielsweise einen Oszillograph zur Darstellung der Kurvenverläufe der Ausgangssignale der beiden Detektoren (28a und 28b) einschließen. Ausgangssignale des Auswerteteils der Schaltung (19) werden weiterhin zur Ansteuerung einer Treiberstufe (10) für die Steuerung des optischen Schalters (10) verwendet.

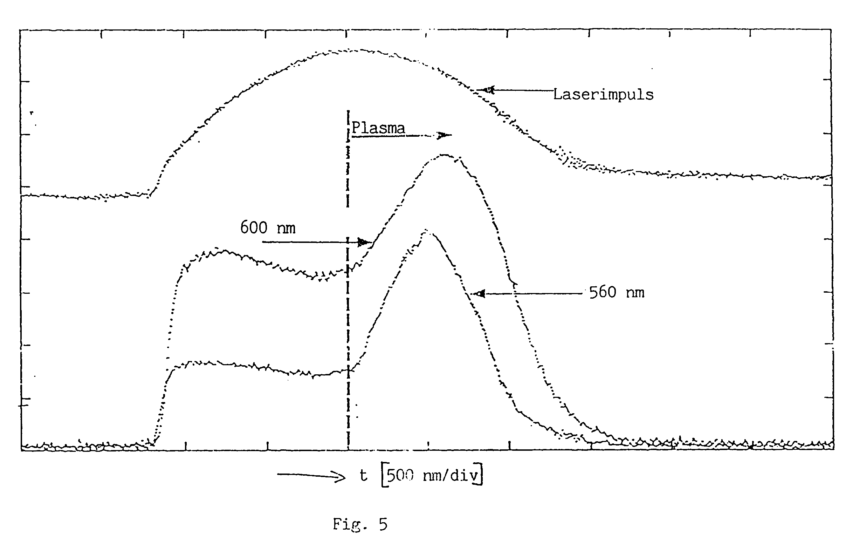

In Fig. 5 ist ein Beispiel für Diagramme dargestellt, die mit dem Anzeigeteil der Auswerteschaltung (19) nach Fig. 4 gewonnen wurden, wobei ein Farbstofflaser mit einer Betriebswellenlänge von 495 nm verwendet wurde. Das Ende des Lichtwellenleiters befand sich in Berührung mit einer Kalkablagerung in einem Blutgefäß. In diesem Fall lag die Laserenergie oberhalb des Schwellwertes für eine Plasmabildung, und dieses Plasma wird ungefähr 1,2 µs nach dem Auslösen des Laserimpulses gezündet. Die Laserenergie betrug hierbei 22 mJ. Unmittelbar zu Beginn des Laserimpulses treten Fluoreszenz-Signale mit einer steilen Anstiegsflanke auf, wobei diese die Intensität des reemittierten Lichtes bei den beiden Durchlaß-Spektralbereichen darstellenden Signale zunächst geringfügig bis zur Plasmazündung absinken. Die Fluoreszenz-Intensität, die durch das reemittierte Licht dargestellt wird, erreicht damit bereits unmittelbar zu Beginn des Laserimpulses ein Maximum, und eine Spektral- oder Intensitäts-Analyse des Fluoreszenz-Lichtes kann damit vor der Zündung des Plasmas durchgeführt werden.

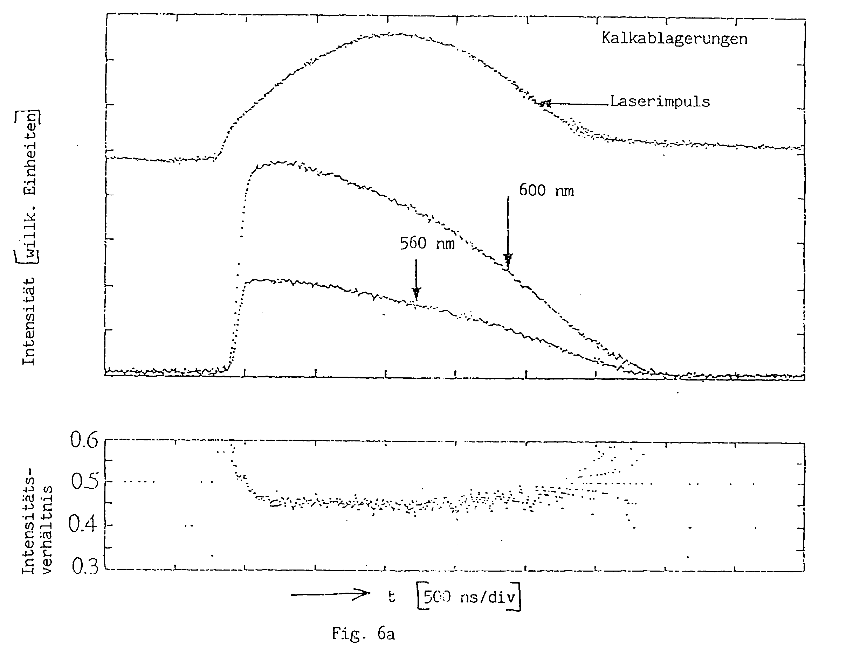

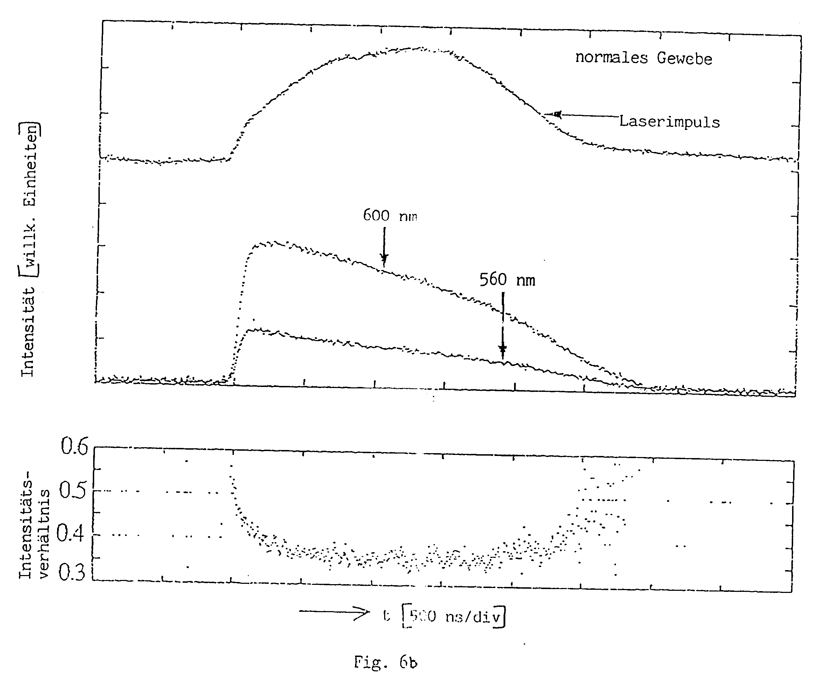

Wenn die Energiedichte des Laserlichtes unterhalb des Plasmaschwellwertes liegt, so ergibt sich der Signalverlauf der Ausgangssignale der beiden Fotodetektoren nach den Fig. 6a bzw. 6b für den Fall von Kalkablagerungen bzw. von normalem Gewebe. Wie aus einem Vergleich der Fig. 6a und 6b zu erkennen ist übersteigt die Intensität des reemittierten, das Fluoreszenz-Licht darstellenden Lichtes für beide Wellenlängen im Fall der Kalkablagerungen nach Fig. 6a wesentlich die Intensität im Fall von normalem Gefäßgewebe nach Fig. 6b. Die absolute Intensität als solche könnte damit bereits als solche zur Unterscheidung zwischen Kalkablagerungen und normalem Gewebe verwendet werden. Damit könnte ein einfacher zeitgesteuerter Vergleicher, der zu einem vorgegebenen Zeitpunkt nach dem Auslösen des Laserimpulses das Ausgangssignal eines der Photodetektoren (28a, 28b) mit einem Bezugssignal vergleicht, zur Feststellung herangezogen werden, ob der Laserimpuls auf Kalkablagerungen oder Gewebe auftrifft. Sowohl an die zeitliche Steuerung als auch an das Bezugssignal wären hierbei jedoch gewisse Anforderungen zu stellen.

Andererseits ist auch aus einem Vergleich der Fig. 6a und 6b in vorteilhafter Weise zu erkennen, daß auch die Verhältnisse der Intensitäten der Signale bei 560 und 600 nm für Kalkablagerungen und normales Gewebe unterschiedlich sind, wobei diese Verhältnisse, wie aus den jeweils unteren Darstellungen der Fig. 6a und 6b zu erkennen ist, bereits ungefähr nach 250 ns einen relativ konstanten Wert erreichen.

Daher kann eine Unterscheidung zwischen Kalkablagerungen und normalem Gewebe durch eine Auswertung des Verhältnisses der Intensitäten in den beiden Spektralbereichen in zuverlässiger Weise erfolgen.

Der genaue Zeitpunkt, zu dem dieser Vergleich durchgeführt wird, ist relativ unkritisch, weil die Intensität des reemittierten Lichtes bei den beiden Spektralbereichen bereits kurz nach dem Einsetzen des Laserimpulses einen Maximalwert erreicht, so daß das Signal-Störverhältnis bereits unmittelbar zu Beginn des Laserimpulses groß ist und genügend Zeit zur Verfügung steht, um den Laserimpuls bei Auftreffen auf normales Gewebe zu unterbrechen.

Dies ist in Fig. 7 dargestellt, in dem der mit Hilfe der Auswerteschaltung gesteuerte Verlauf der Laserenergie für den Fall des Auftreffens auf Kalkablagerungen bzw. normales Gewebe dargestellt ist. Im ersten Fall erfolgt der Verlauf des Laserimpulses ungestört, so daß eine Energie von etwa 30 mJ dem Material vor dem freien Ende des Lichtwellenleiters zugeführt wird, während im zweiten Fall der Laserimpuls unmittelbar nach seinem Auslösen unterbrochen wird, so daß das Gewebe lediglich mit einer Energie von 5 mJ beaufschlagt wird, was weit unterhalb der zum Zünden des Plasmas erforderlichen Energie liegt.

Die elektronische Auswerteschaltung (19) kann einen einfachen Aufbau aufweisen und im wesentlichen ein einstelibares Zeitverzögerungsglied, das beim Auslösen des Laserimpulses gestartet wird, sowie elektronische Rechen- und Vergleicher-Bauelemente einschließen, deren Ausgangssignal sowohl einer Anzeigeeinrichtung als auch der Treiberschaltung (10) zur Steuerung des schnellen optischen Schalters (2) zugeführt wird.

Obwohl im Vorstehenden von einem Farbstofflaser beispielsweise mit einer Betriebswellenlänge von 495 nm und einer Impulsdauer von ungefähr 2 µs ausgegangen wurde, ist es selbstverständlich möglich, die vorstehenden Grundgedanken auch auf andere Laser-Typen mit anderen Betriebswellenlängen und anderen Impulsdauern anzuwenden, die unterschiedliche spektrale Eigenschaften des reemittierten Lichtes für das zu bearbeitende bzw. nicht zu bearbeitende Material hervorrufen. So könnten Festkörperlaser, beispielsweise ein Alexandrit-Laser, gegebenenfalls mit Frequenzverdoppelung verwendet werden.

Weiterhin kann gegebenenfalls die Anzahl der Bandpaßfilter und nachgeschalteten Fotodioden weiter vergrößert werden, um eingehendere Analysen durchzuführen. Wenn die Auswerteschaltung (19) gleichzeitig einen Anzeigeteil enthält, so können die Auswerteergebnisse direkt sichtbar gemacht werden. Durch die Verwendung des Farbhstofffluoreszenz-Sperrfilters (20) vor der Laserlichtquelle kann bei Farbstofflasern die Verwendung eines gespleißten Lichtwellenleiters mit einem zum Laser und einem zur Auswerteoptik führenden Lichtwellenleiterstrang vermieden werden, obwohl auch ein derartig gespeister Lichtwellenleiter unter Fortfall des Filters (20) verwendet werden könnte.

Der Lichtwellenleiter kann in praktischen Anwendungen durch besonders ausgebildete Applikationssysteme, beispielsweise in Form von Wellenleiterbündel-Kathetern gebildet sein, bei denen die einzelnen Wellenleiter aufeinanderfolgeng angesteuert werden bzw. zur Rückleitung des reemittierten Lichtes dienen.

Die anhand der Fig. 4 bis 6 erläuterten Ausführungsformen der Vorrichtung ermöglichen in zuverlässiger und sicherer Weise die Durchführung der Angloplastie ohne die Gefahr einer Schädigung normalen Gewebes.

## Patentansprüche

1. Verfahren zur Materialbearbeitung mit Hilfe eines Lasers, unter Erkennung des zu bearbeitenden Materials, bei dem das Laserlicht über eine Laseroptik auf das Material gerichtet und das von dem Material reemittierte Licht auf eine die Intensität dieses Lichtes messende Detektoranordnung geleitet wird, der eine Auswerteschaltung zur Steuerung des Laserleistung oder -energie nachgeschaltet ist, wobei die Auswerteschaltung für die Steuerung des Lasers und/oder eines im Ausgangsstrahlengang des Lasers angeordneten optischen Schalters den zeitlichen Verlauf der Intensität des auf die Detektoranordnung in einem Spektralbereich einfallenden Lichtes bezogen auf den zeitlichen Verlauf des Laserimpulses in einem vorgegebenen Zeitraum zwischen dessen Auslösung und dem durch ihn hervorgerufenen dielektrischen Durchbruch auswertet und den Laserimpuls vor Erreichen seiner maximalen Energie unterbricht, wenn dieser auf nicht zu bearbeitendes Material auftrifft,
dadurch **gekennzeichnet,** daß zur Bestimmung des von dem Laserimpuls getroffenen Materials und zur Steuerung der Laserleistung oder -energie zu einem vorgegebenen Zeitpunkt in dem vorgegebenen Zeitraum die Intensität des von dem Material reemittierten Lichtes in zumindestens zwei vorgegebenen Spektralbereichen gemessen und ein Quotient der Meßwerte der Lichtenergie in diesen Spektralbereichen gebildet und ausgewertet wird.

2. Verfahren nach Anspruch 1,
dadurch **gekennzeichnet,** daß ein zusätzlicher, im wesentlichen den gesamten Spektralbereich des reemittierten Lichtes erfassender Detektor ein Ausgangssignal zur Normierung des Meßwertes bzw. der Meßwerte der Intensität in dem bzw. den vorgegebenen Spektralbereich(en) verwendet wird.

3. Vorrichtung zur Materialbearbeitung mit Hilfe eines Lasers, dem eine Laseroptik nachgeschaltet ist, die die Laserimpulse auf das zu bearbeitende Material lenkt und von diesem reemittiertes Licht auf eine die Intensität des Lichtes messende Detektoranordnung umlenkt, der eine Auswerteschaltung zur Steuerung des Lasers nachgeschaltet ist, die den zeitlichen Verlauf des auf die Detektoranordnung einfallenden Lichts bezogen auf den zeitlichen Verlauf des Laserimpulses einem vorgegebenen Zeitraum zwischen der Auslösung des Laserimpulses und dem durch diesen hervorgerufenen dielektrischen Durchbruch feststellt und den Laserimpuls vor Erreichen seiner maximalen Energie unterbricht, wenn dieser auf nicht zu bearbeitendes Material auftrifft,
dadurch **gekennzeichnet,** daß die Detektoranordnung (8) mehrere Detektoren umfaßt, die zu einem vorgegebenen Zeitpunkt in dem vorgegebenen Zeitraum auf zumindestens zwei spektrale Bereiche des von dem Material (M) reemittierten Lichtes ansprechen, und daß die Auswerteschaltung eine Rechenschaltung zur Bildung des Quotienten der die Intensität des reemittierten Lichtes in den einzelnen Spektralbereichen darstellenden Ausgangssignale der Detektoren zu dem vorgegebenen Zeitpunkt in dem vorgegebenen Zeitraum einschließt.

4. Vorrichtung nach Anspruch 3,
dadurch **gekennzeichnet,** daß die Laseroptik (3) einen Strahlteiler (4) einschließt, der von dem mit dem Laserimpuls beaufschlagten Material (M) reemittiertes Licht auf die Detektoranordnung (8) umlenkt.

5. Vorrichtung nach Anspruch 3 oder 4,
dadurch **gekennzeichnet**, daß die Laseroptik eine zwischen dem Strahlteiler (4) und der Detektoranordnung (8) angeordnete, die Laserlinie ausblendende Filteranordnung (15) einschließt.

6. Vorrichtung nach einem der Ansprüche 3 bis 5,
dadurch **gekennzeichnet,** daß ein zusätzlicher, im wesentlichen den gesamten Spektralbereich des reemittierten Lichtes erfassender Detektor vorgesehen ist, der ein Ausgangssignal zur Normierung der Meßwerte in einzelnen Spektralbereichen liefert.

7. Vorrichtung nach einem der Ansprüche 3 bis 6,
dadurch **gekennzeichnet,** daß die Rechenschaltung eine Quotienten-Schaltung zur Bildung des Quotienten der Ausgangssignale von zwei oder mehr Detektoren einschließt und das Ausgangssignal der Rechenschaltung einer Steuerschaltung zum Unterbrechen der Laserenergie zugeführt wird.

8. Vorrichtung nach einem der Ansprüche 3 bis 7,
dadurch **gekennzeichnet,** daß die Auswerteschaltung eine Anzeigeschaltung einschließt.

9. Vorrichtung nach einem der Ansprüche 3 bis 8,
dadurch **gekennzeichnet,** daß der Laser ein Farbstofflaser mit einer Betriebswellenlänge im Bereich von etwa 500 nm und einer Impulsdauer im Bereich von etwa 2 µs ist und daß die beiden Spektralbereiche im wesentlichen bei 560 bzw. 600 nm liegen.

10. Vorrichtung nach einem der Ansprüche 7 bis 8,
dadurch **gekennzeichnet,** daß der Laser ein Alexandrit-Laser ist.

11. Vorrichtung nach Anspruch 10,
dadurch **gekennzeichnet,** daß das Ausgangssignal des Alexandrit-Lasers in seiner Frequenz verdoppelt wird.

## Claims

1. A process for treating a material with a laser with identification of the material to be treated, in which the laser light is directed via an optical laser system onto the material and the light re-emitted by the material is conducted onto a detector arrangement, which measures the intensity of said light and which is followed by an evaluating circuit for controlling the laser power or energy, wherein the evaluating circuit evaluates for the control of the laser and/or of an optical switch arranged in the output beam path of the laser the time profile of the intensity of the light incident on the detector arrangement in a spectral range with respect to the time profile of the laser pulse in a predetermined period between the triggering thereof and the dielectric breakdown caused thereby and interrupts the laser pulse before it reaches its maximum energy, when this pulse impinges on material not to be treated,
**characterized** in that
to determine the material struck by the laser pulse and to control the laser power or energy, at a predetermined instant in the the predetermined period, the intensity of the light re-emitted by the material is measured in at least two predetermined spectral ranges and a quotient of the measured values of the light energy insaid spectral ranges is formed and evaluated.

2. A Process according to claim 1,
**characterized** in that an additional detector detecting substantially the entire spectral range of the re-emitted light is employed to generate an output signal for standardizing the measured value or the measured values of the intensity in the predetermined spectral range or ranges.

3. Device for treating a material with a laser which is followed by a laser optical system which directs the laser pulses onto the material to be treated and deflects light re-emitted thereby onto a detector arrangement which measures the intensity of the light and which is followed by an evaluating circuit for the control of the laser, which determines the time profile of the light incident on the detector arrangement with respect to the time profile of the laser pulse in a predetermined period between the triggering of the laser pulse and the dielectric breakdown caused thereby and interrupts the laser pulse before it reaches it maximum energy when said pulse impinges on material not to be treated,
**characterized** in that the detector arrangement (8) includes a plurality of detectors which respond at a predetermined instant in the predetermined period to at least two spectral ranges of the light re-emitted by the material (M) and that the evaluating circuit includes a computing circuit for forming the quotient of the output signals of the detectors representing the intensity of the re-emitted light in the individual spectral ranges at the predetermined instant in the predetermined period.

4. Device according to claim 3,
**characterized** in that the laser optical system (3) includes a beam divider (4), which deflects light re-emitted by the material (M) struck by the laser pulse onto the detector arrangement (8)

5. Device according to claim 3 or 4,
**characterized** in that the laser optical system includes a filter arrangement (15) which is arranged between the beam divider (4) and the detector arrangement (8) and which blocks the laser line.

6. Device according to any one of claims 3 to 5,
**characterized** in that an additional detector is provided which detects substantially the entire spectral range of the re-emitted light and which furnishes an output signal for standardizing the measured values in individual spectral ranges.

7. Device according to any one of claims 3 to 6,
**characterized** in that the computing circuit includes a quotient circuit for forming the quotient of the output signals of two or more detectors and the output signal of the computing circuit is supplied to a control circuit for controlling the laser energy.

8. Device according to any one of the preceding claims,
**characterized** in that the evaluating circuit includes a display circuit.

9. Device according to any one of claims 3 to 8,
**characterized** in that the laser is a dye laser having an operating wave length in the region of about 500 nm and a pulse duration in the region of about 2 µs and that the two spectral ranges lie substantially at 560 and 600 nm.

10. Device according to any one of claims 7 to 8,
**characterized** in that the laser is an alexandrite laser.

11. Device according to claim 10,
**characterized** in that the output signal of the alexandrite laser is doubled in its frequency.

## Revendications

1. Procédé pour le traitement de matières à l'aide d'un laser, reconnaissant la matière à traiter dans laquelle la lumière laser est dirigée par une optique laser sur la matière et la lumière ré-émise par la matière est guidée sur un système de détecteurs mesurant l'intensité de cette lumière, système auquel est monté en aval un circuit d'évaluation pour la commande de la capacité ou énergie du laser, le circuit d'évaluation pour la commande du laser et/ou d'un commutateur optique agencé dans la voie des rayons de sortie du laser évalue le tracé temporel de l'intensité de la lumière frappant le système de détecteurs dans une région spectrale rapporté au tracé temporel de l'impulsion laser dans un laps de temps prédonné entre son déclenchement et le claquage diélectrique provoqué par celui-ci et qui interrompt l'impulsion laser avant d'atteindre son intensité maximale lorsque cette impulsion frappe une matière qui n'est pas à traiter,
caractérisé en ce que pour la détermination de la matière touchée par l'impulsion laser et pour la commande de la capacité ou de l'énergie laser en un instant prédonné dans le laps de temps prédonné, on mesure l'intensité de la lumière ré-émise par la matière dans au moins deux régions spectrales prédonnées et l'on forme un quotient des valeurs mesurées de l'énergie lumineuse dans ces régions spectrales et l'on procède à leur évaluation.

2. Procédé selon la revendication 1, caractérisé en ce que l'on utilise un détecteur supplémentaire, saisissant la lumière ré-émise dans sensiblement la région spectrale globale qui saisit un signal de sortie pour la normalisation de la valeur ou des valeurs mesurées de l'intensité dans la ou dans les régions spectrales prédonnées.

3. Dispositif pour le traitement de matières à l'aide d'un laser auquel est affecté une optique laser qui dirige les impulsions laser sur la matière à traiter et fait dévier la lumière ré-émise par cette matière sur un système de détecteurs mesurant l'intensité de la lumière, auquel est affecté un circuit d'évaluation pour la commande du laser, qui détermine le tracé temporel de la lumière arrivant sur le système de détecteurs rapporté au tracé temporel de l'impulsion laser dans un laps de temps prédonné entre le déclenchement de l'impulsion laser et le claquage diélectri- que provoqué par celui-ci et interrompt l'impulsion laser avant qu'elle n'atteigne son énergie maximale lorsque celui-ci frappe une matière qui n'est pas à traiter,
caractérisé en ce que le système de détecteurs (8) comporte plusieurs détecteurs qui répondent en un instant prédonné dans le laps de temps prédonné à au moins deux régions spectrales de la lumière ré-émise par la matière (M) et en ce que le circuit d'évaluation incorpore un circuit de calcul pour la formation du quotient des signaux de sortie représentant l'intensité de la lumière ré-émise dans les différentes régions spectrales, signaux de sortie des détecteurs à l'instant prédonné dans le laps de temps prédonné.

4. Dispositif selon la revendication 3, caractérisé en ce que l'optique laser (3) incorpore un diviseur de rayons (4) qui fait dévier la lumière ré-émise par la matière (M) frappée par l'impulsion laser sur le système de détecteurs (8).

5. Dispositif selon la revendication 3 ou 4, caractérisé en ce que l'optique laser incorpore un système de filtre (15) qui obture la ligne de laser, agencé entre le diviseur de rayons (4) et le système de détecteurs (8).

6. Dispositif selon l'une des revendications 3 à 5, caractérisé en ce qu'il est prévu un détecteur supplémentaire saisissant sensiblement la totalité de la région spectrale de la lumière ré-émise, détecteur qui fournit un signal de sortie pour la normalisation des valeurs de mesure dans les différentes régions spectrales.

7. Dispositif selon l'une des revendications 3 à 6, caractérisé en ce que le circuit de calcul incorpore un circuit de quotient pour la formation du quotient du signal de sortie de deux ou plus de deux détecteurs et le signal de sortie est amené au circuit de calcul d'un circuit de commande pour l'interruption de l'énergie laser.

8. Dispositif selon l'une des revendications 3 à 7, caractérisé en ce que le circuit d'évaluation incorpore un circuit d'affichage.

9. Dispositif selon l'une des revendications 3 à 8, caractérisé en ce que le laser est un laser à colorant organique accordable avec une longueur d'onde de service dans la plage d'environ 500 nm et une durée d'impulsion dans la plage d'environ 2 µs et en ce que les deux régions spectrales se situent sensiblement à 560 ou 600 nm.

10. Dispositif selon l'une des revendications 7 à 8, caractérisé en ce que le laser est un laser d'Alexandrite.

11. Dispositif selon la revendication 10, caractérisé en ce que le signal de sortie du laser d'Alexandrite est doublé dans sa fréquence.
